# EUROPEAN PATENT APPLICATION

(11) **EP 1 938 699 A1**
(43) Date of publication of application: **02.07.2008**
(21) Application number: 06822023.5
(22) Date of filing: 17.10.2006
(51) Int. Cl.: A23L 1/30, A61K 8/97, A61K 31/07, A61K 36/00, A61P 35/00, C11D 9/00

(54) **EXTRACT LIQUID CONTAINING -CRYPTOXANTHIN INGREDIENT, AND FOOD OR BEVERAGE AND SOAP OR COSMETIC EACH CONTAINING THE EXTRACT LIQUID**

(30) Priority: 20.10.2005 JP 2005305661
(71) Applicant: Toyo Seikan Kaisha, Ltd., Tokyo 100-8522 (JP)
(72) Inventor: TAKAHASHI, Hidehito, Kawanishi-shi, Hyogo 666-0026 (JP); INADA, Yumiko, Kawanishi-shi, Hyogo 666-0026 (JP); KOBAYASHI, Kimie, Kawanishi-shi, Hyogo 666-0026 (JP)
(74) Representative: Jönsson, Hans-Peter
(86) International application number: PCT/JP2006/321002
(87) International publication number: WO 2007/046524

(57) **Abstract**

There are provided an extract solution to be added to food and drink containing a *β*-cryptoxanthin component consisting of an extract containing a *β*-cryptoxanthin component dissolved or dispersed in fat or oil or in a solution comprising an emulsifier and an extract solution to be added to soaps or cosmetics containing a *β*-cryptoxanthin component consisting of an extract containing a *β*-cryptoxanthin component dissolved or dispersed in fat or oil or in a solution comprising an emulsifier. There are also provided food and drink or soaps or cosmetics added with such extract solution containing a *β*-cryptoxanthin component. An extract containing a *β* -cryptoxanthin component derived from persimmon peel is preferable.

## Description

### Technical Field

This invention relates to an extract solution obtained by dissolving or dispersing an extract comprising a *β*-cryptoxanthin component extracted from a persimmon fruit or other fruit in fat or oil or in a solution comprising an emulsifier, and food and drink added with such extract solution. The invention relates also to an extract solution obtained by dissolving or dispersing such extract comprising a β-cryptoxanthin component in fat or oil or in a solution comprising an emulsifier, and soaps and cosmetics added with such extract solution.

### Background Art

*β*-cryptoxanthin which is a kind of carotenoid which has a property of provitamin A and also an anticarcinogenic promoter function and a function to eliminate excessive active oxygen. It has been also found that it is an anticancer substance participating to transmission of information in a cell. Thus, *β*-cryptoxanthin has drawn attention as a material of functional drink and food having these useful functions.

*β*-cryptoxanthin is known to be contained in citrus fruits generally and Japanese Patent Application Laid-open Publication No. 2000-136181 proposes to produce *β*-cryptoxanthin of high purity from an extract by using a solvent containing *β*-cryptoxanthin extracted from a precipitate of a mandarin orange juice.

However, an amount of *β*-cryptoxanthin which is contained in citrus fruits is relatively small and it requires a large amount of raw material to manufacture *β*-cryptoxanthin on the industrial basis which results in increase in cost of raw material and also increase in cost and the number of processes for manufacture.

Japanese Patent Application Laid-open Publication Nos. 2004-329058 and 2004-331528 disclose that it has been found that a much larger amount of *β*-cryptoxanthin is contained in persimmon fruit, particularly persimmon peel than in citrus fruits and that, by extracting *β*-cryptoxanthin from this raw material by using ethanol which is harmless to human body, an extract containing a *β*-cryptoxanthin component can be obtained by a simple process. Thus, these lieterature have opened the way to utilize persimmon peel which has been wasted without being used.

The extract containing a *β*-cryptoxanthin component extracted from persimmon fruit disclosed by Japanese Patent Application Laid-open Publication Nos. 2004-329058 and 2004-331528 is of a highly practical value. As to physical properties of *β*-cryptoxanthin extracted from persimmon fruit, however, no detailed analysis has been practically made in the past. It has been found that when it is attempted to add and distribute this extract in liquefied food such as yogurt, the extract hardly dissolves in water and therefore cannot disperse uniformly in such liquefied food resulting in difficulty in the manufacture of such food. This problem is caused not only in the manufacture of liquefied food but also in a case where the extract containing a *β*-cryptoxanthin component is added and distributed in liquefied soaps or liquefied cosmetics.

### Disclosure of the Invention

The present invention has been made in view of the above described problem arising when an extract containing a *β*-cryptoxanthin component is added and distributed in food and drink or soaps or cosmetics. It is an object of the invention to cause an extract containing a *β*-cryptoxanthin component to dissolve fully and disperse uniformly in food and drink or soaps or cosmetics.

For achieving this object, inventors of the present invention have made laborious studies and experiments which have resulted in the finding, which has led to the present invention, that an extract containing a *β* -cryptoxanthin component extracted from persimmon fruit does not dissolve in water and yet dissolves well in fat or oil or a solution containing an emulsifier and also that, by making an extract solution by dissolving or dispersing the extract containing a *β*-cryptoxanthin component in the fat or oil or the solution containing an emulsifier and adding this extract solution to food and drink or soaps or cosmetics, the extract containing a *β* -cryptoxanthin component is dispersed uniformly in the food and drink or the soaps or cosmetics.

*β*-cryptoxanthin which dissolves in fat and oil cannot be distributed uniformly in water. Hence, by adding a material which has an emulsifying ability to the extract containing a β-cryptoxanthin component, it can be distributed uniformly in water.

As emulsifiers, polyglyceric acid fatty acid ester, phosphoric acid ester and lecithin, e.g., may be used among which polyglyceric acd fatty acid ester is an optimum emulsifier.

An emulsifier to be added to the extract containing a *β*-cryptoxanthin component can be used at a concentration within a range from 0.05% to 10%. A good working efficiency can be obtained by using a solution having a concentration of about 1%. An optimum concentration of *β*-cryptoxanthin in the extract containing a *β*-cryptoxanthin component containing an emulsifier is within a range from 0.1 ppm to 10%.

When it is not necessary to fully distribute the extract containing a *β* -cryptoxanthin component regardless of whether it is orally taken or not, the amount of the emulsifier may be reduced.

For overcoming the above problem, in the first aspect of the invention, there is provided an extract solution containing a *β*-cryptoxanthin component consisting of an extract containing a *β*-cryptoxanthin component dissolved or dispersed in fat or oil or in a solution comprising an emulsifier.

In the second aspect of the invention, there is provided an extract solution containing a *β*-cryptoxanthin component as defined in the first aspect wherein the extract containing a β -cryptoxanthin component is extracted from persimmon fruit.

In the third aspect of the invention, there are provided food and drink added with the extract solution containing a *β*-cryptoxanthin component as defined in the first or second aspect.

In the fourth aspect of the invention, there are provided food and drink as defined in the third aspect comprising calcium.

In the fifth aspect of the invention, there are provided soaps or cosmetics added with the extract solution containing a β-cryptoxanthin component as defined in the first or second aspect.

According to the invention, since the extract containing a *β* -cryptoxanthin component is dissolved or dispersed easily in fat or oil or a solution containing an emulsifier, by first dissolving or dispersing the extract containing a *β*-cryptoxanthin component in fat or oil or a solution containing an emulsifier to form an extract solution and adding this extract solution to food and drink or soaps or cosmetics, the extract containing a β -cryptoxanthin component is dispersed uniformly in food and drink or soaps or cosmetics.

In one aspect of the invention, the advantageous result of the invention can be realized remarkably from the extract containing a β-cryptoxanthin component extracted from persimmon fruit.

In another aspect of the invention, food and drink in which the extract containing a *β*-cryptoxanthin component is uniformly dispersed can be provided.

As such food and drink can be cited, e.g., drinks of various types, cheese, fermented milk, ham and sausage, dressing, yogurt and ace cream.

Such food and drink may be in any form of solid, liquid, gel and jelly.

In another aspect of the invention, the food and drink contain calcium. *β*-cryptoxanthin is known to be effective, in the same manner as vitamin A, for growing osteoblast and preventing osteoporosis. By eating and drinking food and drink containing calcium and being added with *β*-cryptoxanthin and thereby taking *β*-cryptoxanthin and calcium simultaneously, the osteoporosis prevention effect can be further enhanced.

In still another aspect of the invention, soaps or cosmetics in which the extract containing a *β*-cryptoxanthin component is uniformly dispersed can be provided.

These soaps or cosmetics may be in any form of milky lotion, cream, liquid and gel.

### Description of Preferred Embodiments

Embodiments of the invention will now be described.

The present invention is applicable also to an extract containing a ;3 -cryptoxanthin component derived from citrus fruits and other plants. Description will be made below about an extract containing a *β* -cryptoxanthin component derived from persimmon fruit which contains *β* -cryptoxanthin abundantly and therefore has a high practical value and realizes the advantageous result of the present invention remarkably.

In a case of extracting *β*-cryptoxanthin from persimmon fruit, an extract containing a *β*-cryptoxanthin component can be produced by extracting persimmon fruit, preferably persimmon peel, with an organic solvent, particularly ethanol.

There is no limitation in the type of persimmon used as a raw material and an astringent persimmon can be used as well as a sweet persimmon. Persimmon fruit, e.g., fuyu persimmon, contains *β*-cryptoxanthin of 8.36mg/100g in its peel and contains *β*-cryptoxanthin of 0.97mg/100g in its flesh. It is, therefore, preferable to use persimmon peel as a raw material fo producing the extract containing a *β*-cryptoxanthin component but persimmon flesh may also be used.

After peeling persimmon peel, the persimmon peel is cut into pieces with a knife or the like and is crushed by an agitator or the like in the presence of an organic solvent. Then, the persimmon peel is subjected to filtration. The filtrate is concentrated under a reduced pressure and the organic solvent is removed. The filtrate then is rinsed with an organic solvent and concentration is repeated under a reduced pressure until the smell of a dried persimmon disappears to thereby produce the extract containing a β -cryptoxanthin component. In case it is added to food and drink, ethanol should preferably be used as an organic solvent which is harmless to a human body.

Then, the organic solvent is removed from the extract containing a *β* -cryptoxanthin component under a reduced pressure. For removing the organic solvent, a rotary evaporator, e.g., may be used under a reduced pressure to concentrate the extract.

The method for manufacturing the extract containing a *β* -cryptoxanthin component is described in detail by the above described Japanese Patent Application Laid-open Publication Nos. 2004-329058 and 2004-331528.

As a method of extraction other than the one described above, anhydrous sodium sulfate and n-pentane are added to persimmon peel powder the mixture is cooled and vibrated and then is subjected to filtration, concentration and drying. *β*-cryptoxanthin fraction is eluted by the glass column gel chromatography and *β*-cryptoxanthin is crystallized and this crystal is rinsed with water and then filtered to provide *β*-cryptoxanthin.

In case of producing an extract solution of an extract containing a *β* -cryptoxanthin component, the extract containing a β-cryptoxanthin component is dissolved or dispersed in fat or oil or a solution containing an emulsifier.

As fat or oil for dissolving the extract containing a *β*-cryptoxanthin component, any of vegetable oil, animal fat and synthetic fat and oil may be used. As fat and oil which are liquid at room temperature, have good dissolving property and have no smell, a vegetable oil, particularly cottonseed oil, may preferably be used. As to dairy products, milk fat is optimum.

As other solvents for dissolving the extract containing a *β* -cryptoxanthin component can be cited alcohols such as methanol, ethanol, isopropanol and butanol, ethers such as dimethyl ether, diethyl ether, methyl ethyl ether and methyl butyl ether, ketones such as acetone, methyl ethyl ketone and diethyl ketone, and saturated hydrocarbons such as n-hexane and n-heptane.

In case a functional food is produced by adding the extract containing a *β*-cryptoxanthin component to food and drink, concentration of *β* -cryptoxanthin in the food and drink should preferably be in the order of 0.1mg/100g to 50mg/100g. In case it is added to drink, particularly, the concentration should preferably be in the order of 0.1mg/100g to 10mg/100g.

An optimum concentration of the extract containing a *β* -cryptoxanthin component in fat or oil or a solution containing an emulsifier used as a solvent is within a range from 0.1 ppm to 10 weight %. In a case where the amount of *β*-cryptoxanthin may be small, a dilute having *β* -cryptoxanthin of a low concentration is produced and is mixed with food or drink. In a case where *β*-cryptoxanthin is to be added in a high concentration, it is convenient from the standpoint of work efficiency and therefore is preferable to first prepare *β*-cryptoxanthin having a concentration of about 10 weight % and then add it to food and drink.

The invention will be described more fully with reference to examples which in no way restrict the scope of the present invention.

### Example 1

### Production of vanilla ice cream containing β-cryptoxanthin

80g of yolk and 120g of granulated sugar were whipped by a hand mixer to produce a white and smooth batter. Then, 400g of milk which was warmed in a pan by stopping heating immediately before boiling was added little by little and the mixture was stirred with the hand mixer. After stirring, the mixture was shifted to a pan made of stainless steel and was stirred with the hand mixer set at a weak degree of stirring while the mixture was heated gently. When the mixture became smooth, it was cooled with ice water.

Raw cream was prepared in the following manner: 0.001g of an extract containing a *β*-cryptoxanthin component derived from persimmon peel was added to 10g of raw cream and mixed well to dissolve *β* -cryptoxanthin. 90g of raw cream which was whipped by 60% to 80% with the hand mixer was added and mixed and whipped well to prepare 100g of raw cream containing *β*-cryptoxanthin.

A cooled mixture of yolk, granulated sugar and milk was added to this raw cream little by little and the mixture was stirred with the hand mixer. During stirring, vanilla essence was added by 6 shakes. Salt was added and the bowl was put in ice water at a lowered temperature for cooling and the mixture was stirred slowly with the hand mixer for 2 hours. After storing the mixture for 30 minutes in a freezer at -20°C, the mixture was taken out of the freezer and stirred with the hand mixer. The cooling and stirring operations were repeated three times and then the mixture was put in a freezer at - 20°C and 700g of vanilla ice cream containing *β*-cryptoxanthin was provided. In the vanilla ice cream, the extract containing a *β* -cryptoxanthin component derived from persimmon peel was present at a concentration of about 1.4 ppm.

The extract containing a *β*-cryptoxanthin component derived from persimmon peel was dissolved completely in the vanilla ice cream which exhibited a slightly yellow color which was exciting appetite. For comparison, vanilla ice cream was produced in the same manner as in Example 1 except that this ice cream did not contain the extract containing a *β*-cryptoxanthin component and taste of this ice cream was compared with that of Example 1. No difference in taste was found between Example 1 and the comparative example. Milk fat was suitable for dissolving *β* -cryptoxanthin.

Since calcium was contained by 120mg in 80g of yolk, by 440mg in 400g of milk and by 60mg in 100g of raw cream, the total amount of calcium contained in this ice cream was 620mg.

### Example 2

### Production of yogurt containing the extract containing a β-cryptoxanthin component

20ml of raw cream and 0.005g of the extract containing a *β* -cryptoxanthin component derived from persimmon peel were dissolved in a seed bacterium culture dish. After stirring the mixture uniformly, 10ml of milk was added to the mixture. Then, 0.6591g of seed bacterium of yogurt (Caspian yogurt) was added to the mixture and the mixture was stirred with a dispensing spoon. The seed bacterium culture dish was shaken seven times every one hour at room temperature and then was left overnight in a refrigerator at 5°C to provide a seed bacterium called a starter. There was about 40ml of the starter. A portion of 20ml was divided from the starter and shifted to a sterilized glass container. 100ml of raw cream was added to the starter little by little and the mixture was stirred. Then, 100ml of milk was mixed little by little and the mixture was left in the refrigerator at 5°C to provide yogurt containing the extract containing a β-cryptoxanthin component.

In the materials of yogurt, since calcium was contained by 12mg in 20ml of the raw cream containing the seed bacterium, by 12.4mg in 10ml of the milk containing the seed bacterium, by 121mg in 100ml of the additional milk and by 60mg in 100ml of the additional cream, the total amount of calcium contained in this yogurt was 193.2mg.

### Example 3

### Production of soap containing the extract containing a β-cryptoxanthin component

59g of sodium hydroxide was weighed in a beaker in a draft chamber and 175g of ion exchanged water was added. 500g of pork fat was weighed in a bowl made of stainless steel and warmed in warm water under 55°C while it was stirred with a whip to cause the pork fat to dissolve completely. 0.004g of the extract containing a *β*-cryptoxanthin component was added to the dissolved port fat to dissolve *β*-cryptoxanthin. Before and after addition of the extract containing a *β*-cryptoxanthin component, stirring with the whip was continued for about 30 minutes while the temperature of about 55°C was maintained.

Temperature of the sodium hydroxide and that of the pork fat were adjusted to about the same temperature of 42°C. After adjusting of the temperature, the sodium hydroxide was added to the pork fat little by little while the temperature of about 42°C was maintained and stirring with the whip was continued for 30 minutes. Then, while the temperature of about 42°C to 46°C was maintained, stirring for about 1 minute was made every 5 minutes over a period of time of 30 minutes. Then, the mixture was left for about 40 minutes while being warmed at 44°C. Subsequently, stirring for 2 to 3 minutes was made four times every 10 minutes. Since a trace in a spiral form was confirmed in the pork fat when the pork fat was stirred with the whip, the mixture was shifted to an empty milk carton which was then placed in a container made of expandable polystyrene covered in its inside surface with newspaper. The mixture was left for 38 hours while it was kept warmed. Then, solidified soap was taken out of the milk carton and cut into pieces of a proper size and dried for 4 to 6 weeks in the well ventilated shade. Thus, soap containing the extract containing a *β* -cryptoxanthin component was obtained.

### Example 4

As an emulsifier, 10g of 1% solution of DECAGLYN1-OV made by Nihon Surfact Kabushiki Kaisha and sold by Nihon Chemicals Kabushiki Kaisha was warmed to about 40°C and then, while it was gently stirred, 2mg of the extract containing a *β*-cryptoxanthin component was added and dispersed to produce an extract solution containing a *β*-cryptoxanthin component. It was confirmed that 1g of this extract solution was dissolved in 9g of water.

### Industrial Utility

The present invention can be applied to food and drink, or soaps or cosmetics added with an extract solution containing a *β*-cryptoxanthin component.

## Claims

1. An extract solution containing a *β*-cryptoxanthin component consisting of an extract containing a *β*-cryptoxanthin component dissolved or dispersed in fat or oil or in a solution comprising an emulsifier.

2. An extract solution containing a *β*-cryptoxanthin component as defined in claim 1 wherein the extract containing a *β*-cryptoxanthin component is extracted from persimmon fruit.

3. Food and drink added with the extract solution containing a *β* -cryptoxanthin component as defined in claim 1 or 2.

4. Food and drink as defined in claim 3 comprising calcium.

5. Soaps or cosmetics added with the extract solution containing a *β* -cryptoxanthin component as defined in claim 1 or 2.
